# EUROPÄISCHE PATENTANMELDUNG

(11) **EP 1 719 997 A2**
(43) Veröffentlichungstag der Anmeldung: **08.11.2006**
(21) Anmeldenummer: 06008364.9
(22) Anmeldetag: 22.04.2006
(51) Int. Cl.: G01N 1/10

(54) **Verfahren zur Probeentnahme sowie Probeentnahmevorrichtung**

(30) Priorität: 03.05.2005 DE 102005020985
(71) Anmelder: Forschungszentrum Jülich GmbH, 52425 Jülich (DE)
(72) Erfinder: Jockwer, Alexander, 52070 Aachen (DE); Gätgens, Jochem, 52428 Jülich (DE); Noll, Thomas, 52428 Jülich (DE)

(57) **Zusammenfassung**

Die Erfindung betrifft ein Verfahren und eine Vorrichtung zur Entnahme einer Probe aus einem Behälter, wobei die Probeentnahme erfindungsgemäß isobar erfolgen soll.

Durch die isobare Probeentnahme bleibt der pHᵢ-Wert von in der Probe befindlichen Zellen konstant, so dass durch Fluoreszenzmessungen der entnommenen Probe Rückschlüsse auf den physiologischen Zustand der Zellen im Behälter gezogen werden können.

## Beschreibung

Die Erfindung betrifft ein Verfahren zur Probeentnahme sowie eine Probeentnahmevorrichtung.

Bei fermentativen Produktionsprozessen werden Organismen (besonders Mikroorganismen, speziell tierische Zellen) in Bioreaktoren unterschiedlicher Bauart in zumeist flüssigen Medien kultiviert. Hierbei müssen optimale Prozessparameter innerhalb bestimmter Grenzen eingehalten werden, um optimales Wachstum und optimale Produktion zu gewährleisten. Neben der Prozesskontrolle durch in situ installierte Sonden für Parameter wie Gelöstsauerstoff DO und pH-Wert durch die Prozessleitsoftware müssen nach dem Stand der Technik Proben aus dem Bioreaktor entnommen werden, welche die suspendierten Organismen in dem Kulturmedium enthalten. Hierbei müssen die optimalen Prozessparameter so genau wie möglich eingehalten werden. Um eine Kontrolle über den Verlauf der Produktion zu haben, werden nach dem Stand der Technik Proben entnommen, welche Substrat, also die Nährlösung sowie die produzierenden Organismen enthalten. Ein wichtiger Messparameter, aus dem Rückschlüsse über den Zustand der produzierenden Organismen sowie deren Biochemie gezogen werden können, ist der intrazelluläre pH-Wert (pHᵢ). Um den pHᵢ- Wert zu ermitteln, werden nach dem Stand der Technik Fermentationsproben entnommen, indem einfach ein Probevolumen aus dem Fermenter in ein Auffanggefäß abgelassen wird. Nach dem Stand der Technik kann der pHᵢ durch Einbringen eines pH-sensitiven Fluoreszenzfarbstoffes (z. B. Carboxy-SNARF®-1-Acetoxymethylester) in die kultivierten Zellen nach Probeentnahme in ein Auffanggefäß wie folgt bestimmt werden. Zunächst wird innerhalb einer Inkubationszeit (z. B. 25 Minuten) die ausreichende Internalisierung des Farbstoffs in die Zellen gewährleistet (z. B. durch Zugabe des Farbstoffs im polar aprotischen Lösungsmittel Dimethylsulfoxid). Im Innern der Zelle kann der Farbstoff von zelleigenen Mechanismen (z. B. Esterasen) strukturell verändert werden (z. B. Abspaltung der Estergruppe). Dadurch wird er bevorzugt in der Zelle zurückgehalten und ändert pHabhängig seine Fluoreszenzeigenschaften, die man durch geeignete Analytik messen kann (z. B. FACS (= Fluorescence assisted cell sorting = Durchflusszytometrie)). Das Fluoreszenzsignal ist dabei ein empfindlicher Indikator für den pHᵢ₋Wert.

Es ist die Aufgabe der Erfindung, ein Verfahren und eine Vorrichtung zu schaffen, mit denen eine genauere Messung des pHᵢ-Wertes in der Probe möglich ist. Der in der Probe gemessne pHᵢ-Wert soll dabei nicht vom pHᵢ -Wert der Zelle im Fermenter abweichen. In Folge soll die Zellteilung bei der Produktion besser steuerbar sein und es soll die Produktivität der Organismen gesteigert werden.

Ausgehend vom Oberbegriff des Anspruchs 1 wird die Aufgabe überraschenderweise gelöst mit den im kennzeichnenden Teil des Anspruchs angegebenen Merkmalen.
Mit dem erfindungsgemäßen Verfahren und der Vorrichtung ist es nunmehr möglich, den pHᵢ-Wert außerhalb des Fermenters entsprechend den Verhältnissen im Fermenter zu bestimmen. Es ist zudem möglich, präzisere Messwerte für die Gelöstgas-Konzentrationen und die pHᵢ-Werte der kultivierten Zellen in Proben aus drucküberlagerten Bioprozessen zu erhalten. Darauf basierend ist es nunmehr möglich, Gelöst-CO₂- und pHᵢ₋optimierte Bioprozesse hinsichtlich Wachstum und Produktivität der kultivierten Zellen rational zu entwickeln. Weiterhin ist es möglich, durch die präziseren Messwerte für den pHᵢ₋Wert z. B. eine höhere Produktivität und ein geregeltes Wachstum der Zellen zu bewirken. Es hat sich überraschenderweise herausgestellt, dass die Apoptose der durch die erfindungsgemäße Probeentnahme entnommenen Zellen um bis zu 17% im Vergleich zur Probeentnahme nach dem Stand der Technik herabgesetzt wird, so dass die erfindungsgemäße Probeentnahme zellschonender ist. Durch diese Verringerung der Apoptose werden metabolische Parameter besser bestimmt, welche den realen Verhältnissen der Organismen im Fermenter entsprechen. Die Verhältnisse im Fermenter werden daher bei der erfindungsgemäßen Probeentnahme im kleinen Maßstab abgebildet.

Vorteilhafte Weiterbildungen sind in den Unteransprüchen angegeben.

Im Folgenden soll die Erfindung an einem Beispiel erläutert werden.

Die Zeichnung und die Figuren zeigen eine Probeentnahmevorrichtung sowie experimentelle Ergebnisse.

Es zeigt:
- Fig.1:: Eine Probeentnahmevorrichtung
- Fig.2:: Eine Hülse in der Aufsicht und im Querschnitt
- Fig.3:: Vergleich der Messung des pHi-Wertes nach dem Stand der Technik und dem erfindungsgemäßen Verfahren.
- Fig.4a:: Durchflusszytometrische Ergebnisse der Fluoreszenzverhältnisse nach 30 Minuten Inkubation der Fermenterprobe mit dem Farbstoff nach dem St.d.T.
- Fig.4b:: Durchflusszytometrische Ergebnisse der Fluoreszenzverhältnisse nach 60 Minuten Inkubation der Fermenterprobe mit dem Farbstoff nach dem St.d.T.
- Fig.4c:: Durchflusszytometrische Ergebnisse der Fluoreszenzverhältnisse nach 30 Minuten Inkubation der Fermenterprobe mit dem Farbstoff nach dem erfindungsgemäßen Verfahren.
- Fig.4d:: Durchflusszytometrische Ergebnisse der Fluoreszenzverhältnisse nach 30 Minuten Inkubation der Fermenterprobe mit dem Farbstoff nach dem erfindungsgemäßen Verfahren und anschließendem drucklosen Verweilen der Probe für 30 Minuten.
- Fig.5a:: Durchflusszytometrische Ergebnisse des Apoptosestatus nach 30 Minuten Inkubation nach dem Stand der Technik.
- Fig.5b:: Durchflusszytometrische Ergebnisse des Apoptosestatus nach 30 Minuten Inkubation nach dem erfindungsgemäßen Verfahren.

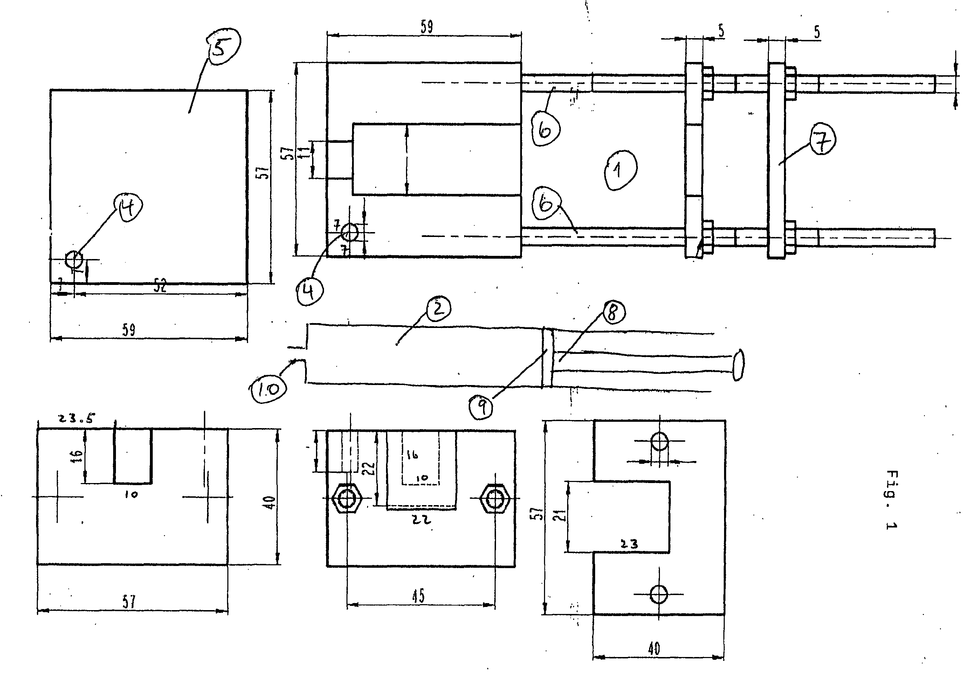

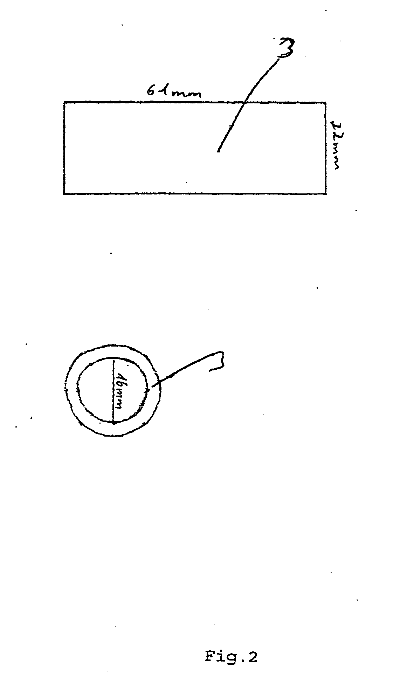

Figur 1 zeigt eine Halterung 1, in welche eine Probeentnahmespritze 2 eingelegt werden kann. Die Probeentnahmespritze 2 kann mit einer Hülse 3 (Fig.2) ummantelt sein, welche die Probeentnahmespritze 2 aufnimmt und welche ein passschlüssiges Einfügen der Probeentnahmespritze 2 in die Halterung 1 ermöglicht. Zur festen Sicherung ist ein mit einer Verschraubung 4 befestigter Deckel 5 an der Halterung 1 angebracht, mit dem die Probeentnahmespritze 2 sicher in der Halterung 1 fixiert werden kann. An zwei Seiten der Halterung 1 befinden sich zwei Schrauben 6, welche in die Halterung 1 eingelassen sind und die eine Wand 7 halten, welche bewirkt, dass der hintere Teil des Stempels 8 der Probeentnahmespritze 2 nicht über einen variabel vorgebbaren, definierten Endpunkt hinausgezogen werden kann. Die Probeentnahmespritze 2 verfügt über einen vorderen Teil des Stempels 9, welcher das aufgenommene Flüssigkeitsvolumen begrenzt sowie einen Anschluss 10, der eine Befestigung an den Probenaustritt ermöglicht.

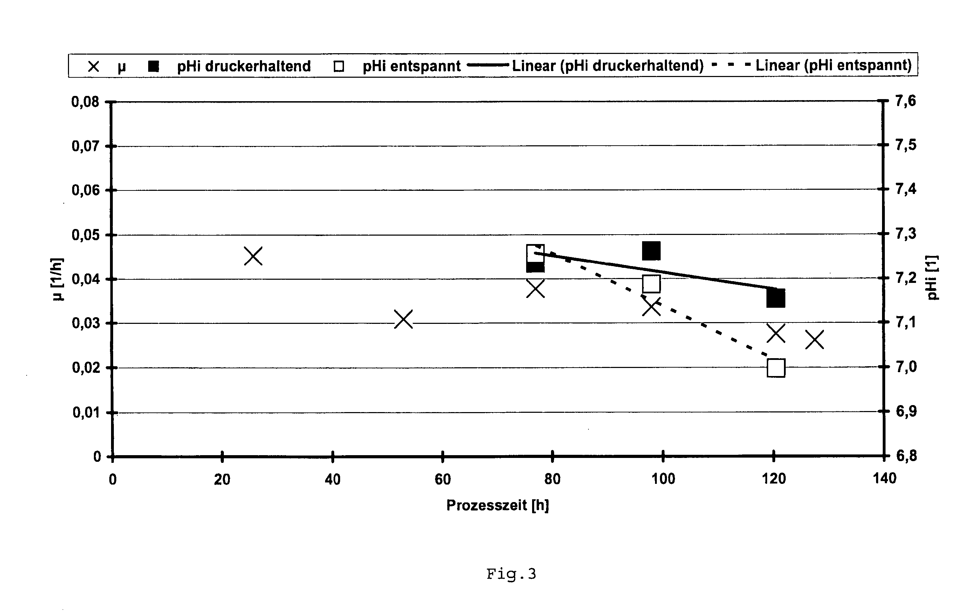

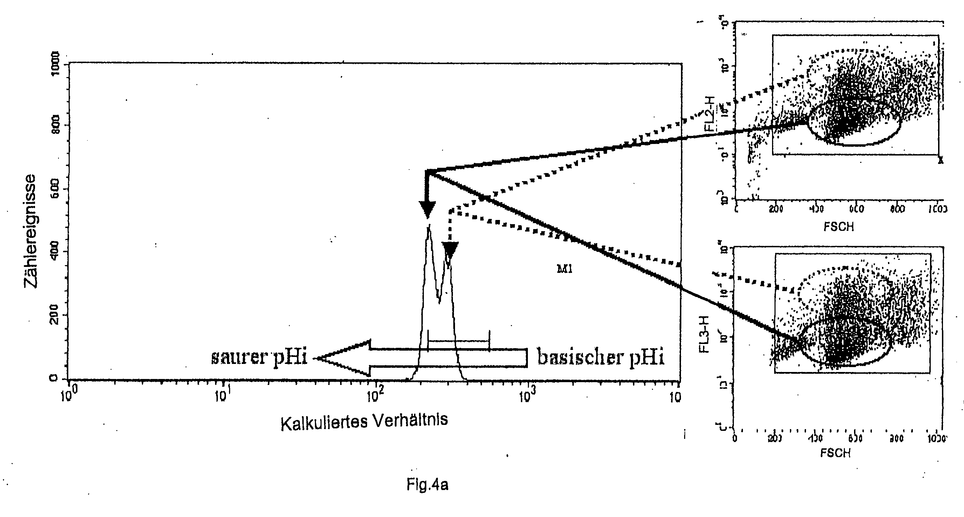

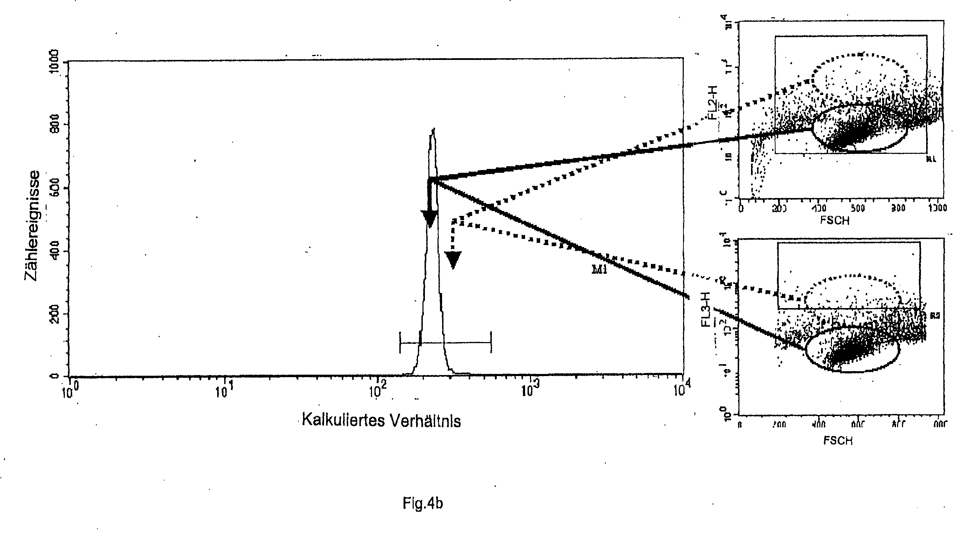

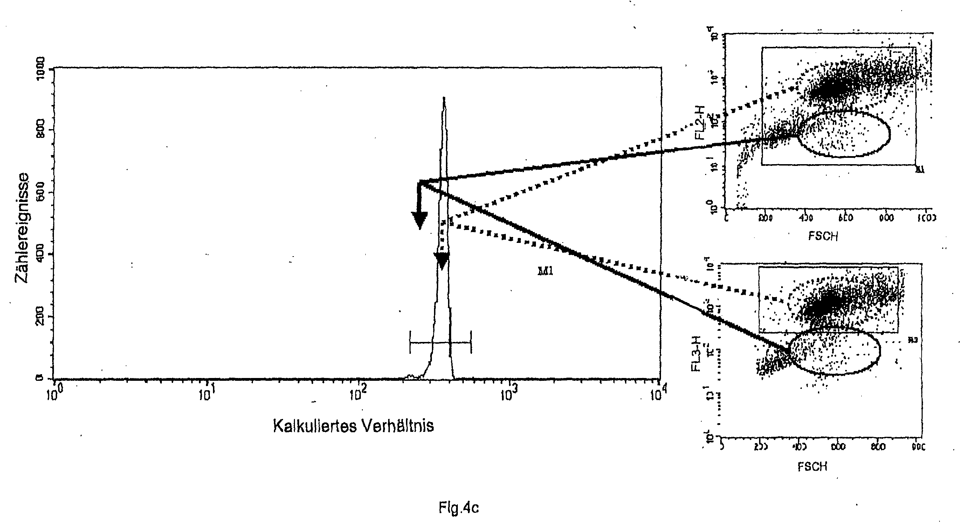

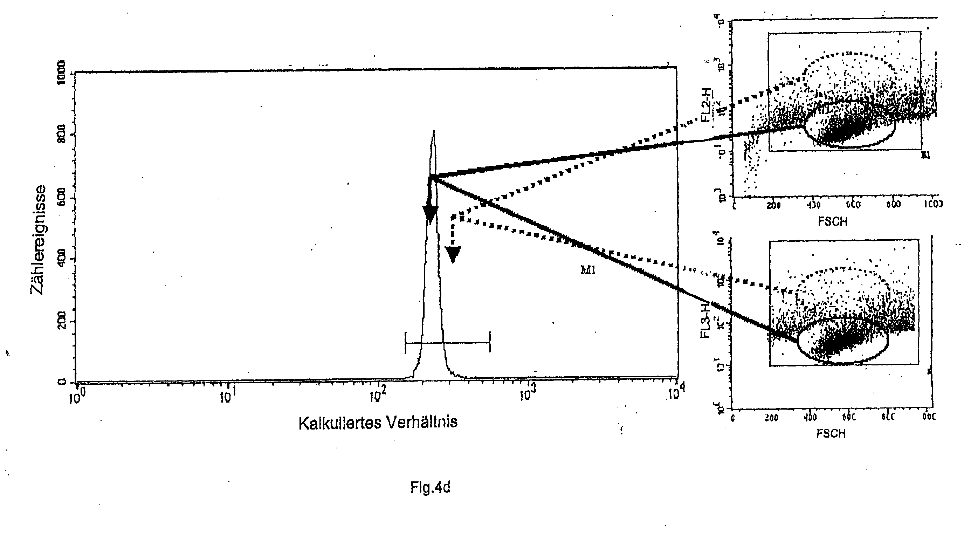

Figur 3 zeigt die mit den unterschiedlichen Probeentnahmearten erhaltenen pHᵢ-Werte zusammen mit der Wachstumsrate µ über der Prozesszeit einer drucküberlagerten CHO-Zell-Fermentation (Überdruck 75 kPa). Die ausgefüllten Kästchen zeigen den zeitlichen Verlauf entsprechend dem erfindungsgemäßen Verfahren, die leeren Kästchen den Verlauf nach dem Stand der Technik. Die Unterschiede in den Messergebnissen werden durch die Fig. 4 a - d erläutert.
Figur 4 a zeigt Ergebnisse der Durchflusszytometrie (FACS), die zur Ermittlung des pHᵢ-Wertes der Fementationsprobe (CHO-Zelllinie aus drucküberlagertem Fermenter) analog Cherlet et al. (Cherlet et al., Biotechnol. Prog. 1999, 15, 630-639) nach dem Stand der Technik mit dem Fluoreszenzfarbstoff SNARF-1-AM (Molecular Probes, USA) nach dreißig Minuten Inkubation bei Atmosphären-Druck mit der pseudo-0-Kalibrationsmethode (Becton-Dickinson) erzielt wurden. Der in der linken Darstellung auftretende Doppelpeak bei Auftragung der Zählereignisse (Counts) gegen das Fluoreszenzintensitätsverhältnis (Ratio) der pH-abhängigen Emissionsmaxima (hier FL3 bzw. FL2, Fig. 4 a rechts unten bzw. rechts oben) des eingesetzten Farbstoffs resultiert aus einer entspannungszeitabhängigen, heterogenen Veränderung des Fluoreszenzverhaltens der untersuchten Zellpopulation nach der Probeentnahme nach dem Stand der Technik. In Figur 4 a rechts sind für beide detektierten Fluoreszenzen zwei Hauptpopulationen erkennbar (Ellipsen). Die entspannungszeitabhängige Veränderung der Population wird nach sechzig Minuten Inkubation derselben Probe analog in Fig. 4 b dargestellt. Hier befindet sich die gesamte Population in Richtung saurerer pHᵢ verschoben, die Heterogenität ist aufgehoben. Bei ausschließlicher Kenntnis des Vorhandenseins des linken Einzelpeaks würde der ermittelte pHᵢ-Wert nicht dem pHᵢ-Wert der Zellen im Reaktor entsprechen.
Bei analoger Bestimmung des pHᵢ-Wertes der Zellen durch FACS zeigen die Zellen nach erfindungsgemäßer drucküberlagerter Probeentnahme auch nach 30-minütiger Inkubation eine Einzelpopulation, die demnach den Verhältnissen im Fermenter entsprechen muss. Dies ist in Figur 4c dargestellt. Auch hier führt eine nachträgliche Entspannung derselben Probe und weiterer Inkubation bei Atmosphären-Druck wie bei der konventionellen Probeninkubation zu einer homogen veränderten Einzelpopulation gleichen Fluoreszenzintensitätsverhältnisses (Fig. 4 d) .

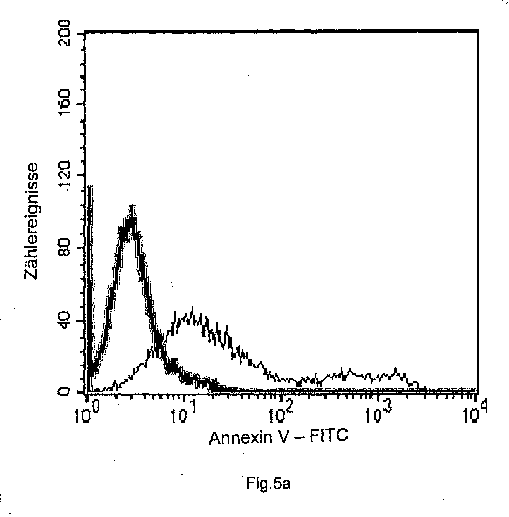

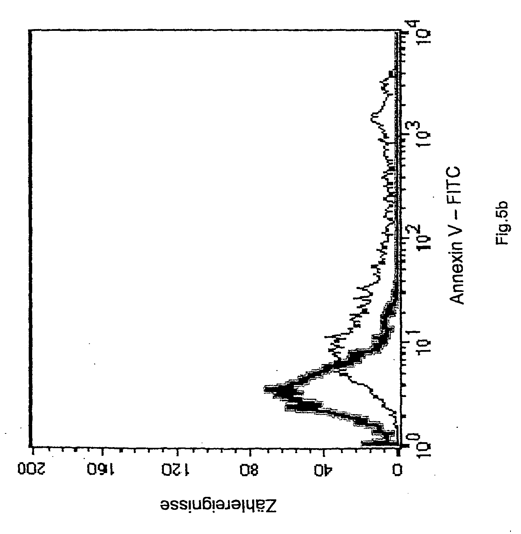

Fig. 5a zeigt die durchflusszytometrischen Ergebnisse einer Annexin-V-FITC gefärbten CHO-Zell-Probe, die einem drucküberlagerten Fermenter nach herkömmlicher Art entnommen wurde. Die Externalisierung von Phosphatidylserin dient als früher Apoptosemarker, woran Annexin-V-FITC binden kann und dann als Fluoreszenzsignal im FACS erkannt wird. Diese Fluoreszenzintensität ist in Fig. 5a auf der x-Achse aufgetragen. Die y-Achse zeigt die zugehörigen Zählereignisse. Der fett gezeichnete Kurvenverlauf links in Fig. 5a ergibt sich aus der ungefärbten Zellsuspension (Negativprobe). Färbung der entnommenen Zellen nach herkömmlicher Probeentnahmeart resultiert in der dünn gezeichneten Kurve, rechts gezeigt in Fig. 5 a. Dies entspricht einer Zunahme von 62%.
Bei Verwendung der erfindungsgemäßen Probeentnahmevorrichtung liegt dieser Anstieg bei 45%, also absolut 17% geringer (Fig. 5b).

Bei dem Verfahren werden Organismen, Zellen oder Mikroorganismen in einem Fermenter zur Produktion von Chemikalien oder Zellen herangezogen. Die Produktionsorganismen befinden sich dabei in einem für die Fermentation geeigneten flüssigen, gasförmigen oder festen Medium. Als festes Medium kann eine Trägersubstanz verwendet werden, auf der die Organismen immobilisiert sind und die sich in einer flüssigen oder Gasphase befindet.

Als Organismen können Prokaryonten, Eukaryonten oder beispielsweise Pilze eingesetzt werden.

Bei der Fermentation können Zellen, wie Stammzellen, Blutstammzellen, Immunzellen, Hautzellen oder beispielsweise Leberzellen sowie immortalisierte Zelllinien gezüchtet werden. Die Aufzählung ist aber nicht beschränkend.

Als stoffliche Produkte können beispielsweise, aber nicht beschränkend, monoklonale Antikörper, biologische Substanzen, wie rekombinante Proteine oder Wachstumsfaktoren genannt werden. Es ist aber auch die Produktion von Feinchemikalien, wie Aminosäuren möglich. Auch hier ist die Aufzählung nicht beschränkend.

Das Medium ist eine wässrige Phase, die neben den Produktionszellen zusätzliche Komponenten enthält.

Der pH-Wert des Mediums liegt beispielsweise in einer Größenordnung von pH 2-10, bevorzugt physiologisch 6,5-7,5 und hängt vom Organismus ab.

Für die Einstellung des pH-Wertes geeignete Puffer sind beispielsweise Hydrogencarbonat, HEPES oder MOPS.

Als Nährstoffe können beispielsweise Glukose, Laktat, Glutamin, Aminosäuren, Zucker oder Intermediate des Stoffwechsels eingesetzt werden.

Weitere Komponenten können beispielsweise Mineralien, Spurenelemente, Vitamine, Peptone, Oligopeptide, Hydrolysate von Eiweißen pflanzlicher oder tierischer Herkunft sein.

Die Prozesstemperatur liegt beispielsweise in einem Bereich von -20°C-120°C, vorzugsweise bei 0°C-60°C, besonders bevorzugt zwischen 15°C-45°C.

Vorzugsweise wird die Probeentnahme isotherm durchgeführt.

Die Drucke in dem Fermenter betragen üblicherweise zwischen 900 und 20000 hPa, vorzugsweise zwischen 900-5000 hPa besonders bevorzugt zwischen 1050-2000 hPa und hängen von den Bedürfnissen des Organismus und den verfahrensbedingten Rahmenbedingungen ab.

Die die Zielverbindung produzierenden Organismen scheiden das Produkt und Stoffwechselprodukte aus. Als aerobes Atmungsprodukt wird CO₂ aus der Zelle exportiert. Damit verbunden ist eine gleichgewichtsbedingte CO₂-Konzentration in der produzierenden Zelle, welche Einfluss auf den Stoffwechsel der Zelle nimmt.
Die durch die adhärent oder in Suspension kultivierten Zellen produzierte Zielverbindung wird von den Zellen entweder in das Kulturmedium sekretiert oder reichert sich in den Zellen an. Auch können die Zellen selbst das Produkt der Kultivierung sein. In jedem Fall produzieren die kultivierten Zellen als Stoffwechselprodukt CO₂, welches selbst kultivierungsbedingt im Nährmedium von der für die kultivierten Zellen optimalen Konzentration abweichen kann.

Ein wichtiger Prozess-Parameter, der während des Bioprozesses innerhalb bestimmter physiologischer Konzentrationsgrenzen gehalten werden muss, ist daher die CO₂-Konzentration im Nährmedium. Das von den Organismen produzierte und in Bioprozessen zur pH-Korrektur eingesetzte CO₂ befindet sich in wässrigen Lösungen in folgendem pH-abhängigen Gleichgewicht:

CO₂ (g) + H₂O (1) ↔ H₂CO₃ (aq) ↔ H⁺ + HCO₃⁻ ↔ 2H⁺ + CO₃²⁻

Somit findet bei Druckminderung gemäß dem Henry schen Gesetz in der entnommenen Zellsuspension durch Probeentnahme nach dem Stand der Technik auch eine Verlagerung des CO₂-Gleichgewichts und des pH-Wertes (H⁺-Konzentration) statt. Da CO₂ als unpolares kleines Molekül die Organismen ungehindert passieren kann, findet diese Gleichgewichtsverlagerung bei Probeentnahme nach dem Stand der Technik auch im Inneren der Zellen statt. Daran gekoppelt ist, wie auch im umgebenden Nährmedium, eine Änderung des pH-Werts im Inneren der im entnommenen Probevolumen befindlichen Zellen (pHᵢ).
Weiterhin können alle Gleichgewichte die metabolischen Messungen beeinflussen, die druck- und/oder protonenabhängig sind. Beispielsweise können Stoffe, wie Ammoniak oder leicht flüchtige Säuren wie z. B. Butyrat bzw. Buttersäure Einfluss auf den pHᵢ-Wert nehmen.
Die durch Entspannung der Suspension bewirkte CO₂-Gleichgewichtsverschiebung beeinflusst somit maßgeblich das Ergebnis der Messung des intrazellulären pH-Werts, einem wichtigen biochemischen Parameter, über den prozessrelevante Informationen über den metabolischen Zustand der Zelle zugänglich sind. So ist der pHᵢ-Wert z. B. in den verschiedenen Zellzyklusphasen erheblich unterschiedlich. Es können Rückschlüsse auf den physiologischen Zustand der Zellen gezogen werden.
Um die Prozessparameter im Hinblick auf die Raum-Zeit-Ausbeuten der Produkte und/oder im Hinblick auf ein angestrebtes Wachstum der kultivierten Zellen im Bioreaktor zu optimieren, wird der pHᵢ-Wert als Parameter während der Kultivierung gemessen.

Erfindungsgemäß wird der pHᵢ- Wert nun dadurch bestimmt, dass eine Fermentationsprobe im Wesentlichen isobar entnommen und mit Mitteln zur Bestimmung des pHᵢ-Wertes vermessen wird. Hierdurch wird nicht nur die Messungenauigkeit im Hinblick auf die im Fermenter vorliegenden Verhältnisse verbessert, sondern es wird auch zusätzlich die Apoptose eingeschränkt.

Unter im Wesentlichen isobar im Sinne der Erfindung ist zu verstehen, dass bei der Probeentnahme und beispielsweise während des Mischens der Probe mit dem Fluoreszenzfarbstoff kein Druckabfall im Probevolumen auftritt, welcher durch Verlagerung des chemischen CO₂-Gleichgewichts (s.o.) eine Änderung des pH-Werts im Kulturmedium, wie auch innerhalb der Zelle bewirken würde und somit der pHᵢ der entnommenen Zellen nicht dem pHᵢ der im Bioreaktor befindlichen Zellen entsprechen würde. Vorzugsweise soll die Genauigkeit so groß sein, dass die Messergebnisse für den pHᵢ-Wert durch Fluoreszenzmessung für die Bestimmung in einem Fermenter die gleichen Ergebnisse liefert, wie außerhalb des Fermenters.

Bei der erfindungsgemäßen Probeentnahme ist die Probeentnahmespritze 2 entweder direkt oder über eine Zwischenleitung an den Fermenter angeschlossen. Die erfindungsgemäße isobare Probeentnahme kann auf verschiedene Weise erfolgen.

Im Folgenden wird von dem Begriff der Probeentnahmespritze Gebrauch gemacht. In Sinne der Erfindung ist damit aber jedes Auffangbehältnis umfasst, welches eine isobare Probeentnahme ermöglicht, auch wenn es nicht die Form einer Spritze besitzt.

In einer einfachen Ausführungsform nimmt die Probeentnahmespritze 2 das aus dem Fermenter austretende Flüssigkeitsvolumen auf. Dabei wird keine Flüssigkeit aus dem Reaktor gepumpt oder gesaugt, vielmehr bewegt in einer bevorzugten Ausführungsform die austretende Flüssigkeit den vorderen Teil 9 des Stempels, was zu einer isobaren Entnahme der Flüssigkeit führt.
Alternativ ist es denkbar, den Stempel 9 der Probeentnahmespritze 2 so langsam herauszuziehen, dass in der Spritze faktisch kein Druckunterschied zum Druck im Fermenter entsteht. Die isobare Ausdehnung des Stempels 9 auf Grund der austretenden Flüssigkeit ist aber jedenfalls eine sichere Methode, den Prozess der Probeentnahme isobar verlaufen zu lassen. An Stelle der Schraube 6 kann daher auch eine Schiene treten, welche ein Herausgleiten des Stempels 9 ermöglicht.

In einer weiteren möglichen Ausführungsform ist die Vorrichtung zur Probeentnahme mit einem Motor ausgestattet, welcher den Stempel 7 der Probeentnahmespritze 2 mit einer Geschwindigkeit nach hinten zieht, welche zu einem isobaren Flüssigkeitsaustritt aus dem Fermenter führt.

In einer Weiterbildung der Erfindung besitzt der Fermenter einen Sensor, welcher den Innendruck des Fermenters bestimmt. Vorzugsweise steht der Drucksensor im Fermenter mit dem Druckmessgerät, welches den Druck in der Leitung oder in der Probeentnahmespritze 2 misst über eine Steuerung in Verbindung, welche die Geschwindigkeit, mit der die Probe entnommen wird, in Anhängigkeit von der möglichen Druckdifferenz steuert.

Diese Möglichkeiten sind Beispiele für Mittel zur isobaren Entnahme einer Probe.

Zur Probeentnahme kann die Probeentnahmespritze 2 direkt oder durch Verbindungsleitungen am Fermenter angebracht sein. Der Anschluss 10 der Probeentnahmespritze 2 kann mit einem Rückschlagventil ausgestattet sein. Der Probenaustritt kann ebenfalls mit einem Ventil ausgestattet sein.

An der Probeentnahmespritze 2 oder an einer Zuleitung zwischen Fermenter und Probeentnahmespritze 2 können Messgeräte, insbesondere Druckmessgeräte, angebracht sein, welche die Kontrolle ermöglichen, ob die physikalischen und chemischen Eigenschaften in dem aus dem Reaktor austretenden Volumen mit denen der Prozessflüssigkeit im Reaktor übereinstimmen.

Die Mittel zum Messen des pHᵢ-Wertes können ein Fluoreszenzfarbstoff sein, welcher in das Aufnahmegefäß vorgelegt wurde oder von den Organismen selbst hergestellt wird, eine MikropH-Elektrode, die an eine einzelne Zelle angebracht wird, aber auch ein NMR-Gerät, welches Signale liefert, die mit dem pH-Wert korrelieren, beispielsweise Phosphor- oder H-NMR. Im Falle der NMR-Messung muss ein über die gesamte Probe gemitteltes Messsignal z. B. an Hand einer Eichkurve zur Auswertung herangezogen werden. Als Lumineszenzfarbstoff können Fluoreszenz- oder Phosphoreszenzfarbstoffe eingesetzt werden. Für die Gruppe der Fluoreszenzfarbstoffe können beispielhaft folgende Stoffe genannt werden:

### Liste Fluoreszenzfarbstoffe:

Hersteller Molecular Probes Europe BV, Leiden, The Netherlands
- 1.: SNARF Indikatoren
- 2.: SNAFL Indikatoren
- 3.: HPTS (Pyranin)
- 4.: BCECF
- 5.: Fluoresceine und Carboxyfluoresceine
- 6.: LysoSensor Green DND-189
- 7.: Oregon Green Farbstoffe
- 8.: LysoSensor Yellow/Blue DND-160

Die Spritze bzw. das Aufnahmegefäß ist vorzugsweise gasdicht ausgebildet. Hierzu dienen beispielsweise ein Rückschlagventil oder eine Klemme an der Spitze der Probeentnahmespritze 2 oder der Zuleitung.

Analog zur erfindungsgemäßen Probeentnahmevorrichtung ist auch der Entnahmestutzen am Reaktor und/oder an der Entnahmeleitung vorzugsweise mit einem Ventil ausgestattet, so dass keine Fremdstoffe oder Organismen in den Fermenter gelangen können und dort Einfluss auf den Fermentationsprozess nehmen.

Im weiteren Sinne kann an Stelle eines Fermenters auch ein anderer Reaktor treten, aus dem eine Probe entnommen wird, um den pH-Wert einer Lösung den tatsächlichen Verhältnissen entsprechend genau zu messen. Dies kann beispielsweise bei der chemischen Prozessführung bei Reaktionen, die empfindlich vom pH-Wert abhängen, beispielsweise Enzymreaktionen, sinnvoll sein. Die Probeentnahmevorrichtung muss in diesem Fall an den Prozess angepasst sein. Dies kann beispielsweise durch Verwendung von inerten Materialien für die Aufnahme der Probe geschehen.

In der Probeentnahmespritze 2 oder allgemein im Aufnahmebehälter ist der zur Analyse dienende Fluoreszenzfarbstoff vorgelegt, welcher über einen Zeitraum von ca. 25 Minuten in die Produktionszellen aufgenommen wird. Ist der Fluoreszenzfarbstoff in der Zelle, so kann die Fermenterprobe in bekannter Weise der Fluoreszenzanalyse zugeführt werden, und zwar indem die Probe aus der Probeentnahmespritze 2 in die normale Umgebung abgegeben wird.

Nach der Verweilzeit, die die Probe in der Spritze verbleiben muss, damit der Fluoreszenzfarbstoff, welcher der Analyse dient, in die Zellen aufgenommen werden kann, kann die Probe der Messung zugeführt werden.

Es hat sich nunmehr überraschenderweise herausgestellt, dass die durch die erfindungsgemäße Probeentnahme gemessenen pHᵢ₋Werte denen im Fermenter entsprechen.

Durch die Probeentnahme, wie sie an größeren Fermentern (>11) praktiziert wird (Entspannung des Reaktors durch Belassen der Zellsuspension in atmosphärischen Druck), findet bereits eine wesentliche Entgasung der Reaktorsuspension statt. Diese verschiebt das Löslichkeitsgleichgewicht für CO₂, so dass die Entspannung der Lösung mit einer Verringerung der HCO₃⁻ Pufferkapazität einhergeht und so der pH-Wert der Lösung steigt. Die Auswirkung der Probeentnahme spiegelt sich zunächst hauptsächlich somit auch im intrazellulären pH-Wert, einem wichtigen metabolischen Parameter der Zellen wieder. Bevor diese externe Änderung messbar wird, ändert sich zunächst der intrazelluläre pH-Wert, abhängig vom Druck und somit gelöst-CO₂-Unterschied innerhalb der Zelle. Daher ist das erfindungsgemäße Verfahren besonders bei Fermentern mit großer Füllhöhe vorteilhaft. Die Zugabe von festen, gasförmigen oder flüssigen Substanzen - z. B. Farbstoff- oder Antikörperlösungen - vor, während oder nach der Probeentnahme zur biochemischen Untersuchung der Zelle unter Bioreaktorbedingungen, ist ebenfalls möglich. Die erfindungsgemäße druckhaltende Probeentnahme erlaubt es aus Bioreaktoren sterile, in ihrer Zusammensetzung bezüglich Gelöstgasen dem Reaktorinhalt entsprechende Proben zu entnehmen. Darüber hinaus bietet sie die Möglichkeit über einen Sensor den aktuellen Druck der Probe anzuzeigen und falls nötig, diesen zu regulieren. In Verbindung mit Blutgasanalysatoren ist eine genaue Quantifizierung der Gelöstgase auch nach längeren Zeitintervallen und Behandlungsschritten in der Probensuspension möglich. Das Verfahren ist natürlich auch mit Zellen anwendbar, welche über natürliche Fluoreszenzstoffe als natürliche Zellinhaltsstoffe verfügen, die als Messindikatoren dienen können.

Das erfindungsgemäße Verfahren ist grundsätzlich für alle z. B. chemischen Produktionsverfahren geeignet, deren Ausbeuten und Qualitäten vom pH-Wert und/oder der Gelöstgaskonzentration abhängen.

## Patentansprüche

1. Verfahren zur Probeentnahme, bei dem aus einem Behälter ein Flüssigkeitsvolumen in einen Aufnahmebehälter entnommen wird,
**dadurch gekennzeichnet,**
**dass** die Probeentnahme isobar erfolgt.

2. Verfahren nach Anspruch 1,
**dadurch gekennzeichnet,**
**dass** die Entnahme durch Austritt aus dem Behälter gegen einen Stempel erfolgt.

3. Verfahren nach Anspruch 2,
**dadurch gekennzeichnet,**
**dass** die Entnahme durch Austritt aus dem Behälter gegen einen Stempel erfolgt, welcher mit einer Geschwindigkeit herausgezogen wird, welche eine isobare Entnahme ermöglicht.

4. Verfahren nach einem der Ansprüche 1 bis 3,
**dadurch gekennzeichnet,**
**dass** der Druck im Aufnahmebehälter gemessen und mit dem Druck des Behälters, dem die Probe entnommen wird, verglichen wird.

5. Verfahren nach Anspruch 4,
**dadurch gekennzeichnet,**
**dass** die Geschwindigkeit der Entnahme der Probe bei Abweichung des Druckes im Behälter, aus dem die Probe entnommen wird und dem Aufnahmebehälter so angepasst wird, dass der Innendruck des Aufnahmebehälters und des Behälters, dem die Probe entnommen wird, gleich ist.

6. Verfahren nach einem der Ansprüche 1 bis 5,
**dadurch gekennzeichnet,**
**dass** die Probe Zellen, Organismen oder Mikroorganismen enthält.

7. Verfahren nach Anspruch 6,
**dadurch gekennzeichnet,**
**dass** die Zellen der Probe Zellen produzieren.

8. Verfahren nach Anspruch 6,
**dadurch gekennzeichnet,**
**dass** die Zellen der Probe monoklonale Antikörper, rekombinante Proteine, Aminosäuren, Feinchemikalien oder Wachstumsfaktoren produzieren.

9. Verfahren nach einem der Ansprüche 1 bis 8,
**dadurch gekennzeichnet,**
**dass** der Druck der Probe zwischen 900 und 20000 hPa liegt.

10. Verfahren nach einem der Ansprüche 1 bis 9,
**dadurch gekennzeichnet,**
**dass** die entnommene Probe einer pHᵢ-Wert Messung zugeführt wird.

11. Verfahren nach Anspruch 10,
**dadurch gekennzeichnet,**
**dass** die Probe zwischen der Entnahme und der pHᵢ- Messung wenigstens 10 Minuten lagert.

12. Verfahren nach Anspruch 10 oder 11,
**dadurch gekennzeichnet,**
**dass** der pHᵢ- Wert durch Fluoreszenzanalyse, eine Mikroelektrode oder ein NMR-Gerät bestimmt wird.

13. Verfahren nach einem der Ansprüche 1 bis 12,
**dadurch gekennzeichnet,**
**dass** die Probeentnahme isotherm erfolgt.

14. Vorrichtung zur Durchführung des Verfahrens nach einem der Ansprüche 1 bis 12,
**dadurch gekennzeichnet,**
**dass** sie ein Aufnahmegefäß und Mittel zur isobaren Entnahme einer Probe aus einem Behälter umfasst.

15. Vorrichtung nach Anspruch 14,
**dadurch gekennzeichnet,**
**dass** das Aufnahmegefäß eine Probeentnahmespritze (2) ist.

16. Vorrichtung nach Anspruch 1 oder 2,
**dadurch gekennzeichnet,**
**dass** sie einen Stempel (8) umfasst, welcher ein Eintreten eines Mediums ermöglicht.

17. Vorrichtung nach Anspruch 16,
**dadurch gekennzeichnet,**
**dass** sie über mindestens eine Schraube (6) verfügt, welche mit einer Halterung (1) des Aufnahmegefäßes verbunden ist, wobei die Schraube (6) die Regulierung eines isobaren Volumeneintritts einer Flüssigkeit in das Aufnahmegefäß ermöglicht.

18. Vorrichtung nach einem der Ansprüche 14 bis 17,
**dadurch gekennzeichnet,**
**dass** sie über einen Motor verfügt, welcher einen Stempel mit einer Geschwindigkeit nach hinten zieht, welche einen isobaren Flüssigkeitsaustritt aus einem Fermenter ermöglicht.

19. Vorrichtung nach einem der Ansprüche 1 bis 18,
**dadurch gekennzeichnet,**
**dass** das Auffanggefäß einen Druckmesser beinhaltet.

20. Vorrichtung nach einem der Ansprüche 1 bis 19,
**dadurch gekennzeichnet,**
**dass** sie über einen Sensor verfügt, welcher den Innendruck eines Produktionsreaktors misst, aus dem die Probe entnommen wird.

21. Vorrichtung nach Anspruch 20,
**dadurch gekennzeichnet,**
**dass** sie über eine Steuerung verfügt, welche den Innendruck des Aufnahmegefäßes in Abhängigkeit von dem im Produktionsreaktor gemessenen Innendruck abgleicht.

22. Vorrichtung nach einem der Ansprüche 14 bis 21,
**dadurch gekennzeichnet,**
**dass** das Aufnahmegefäß durch eine Leitung mit einem Produktionsreaktor in Verbindung steht.

23. Vorrichtung nach einem der Ansprüche 14 bis 22,
**dadurch gekennzeichnet,**
**dass** das Aufnahmegefäß über einen Anschluss 10 mit einem Rückschlagventil verfügt.
